# EUROPEAN PATENT APPLICATION

(11) **EP 2 158 919 A1**
(43) Date of publication of application: **03.03.2010**
(21) Application number: 09168876.2
(22) Date of filing: 27.08.2009
(51) Int. Cl.: A61K 38/01, A61K 35/20, A61P 3/08

(54) **Method for reducing postprandial glucose levels with a composition of whey protein and fiber**

(30) Priority: 29.08.2008 US 93037 P
(71) Applicant: Kraft Foods Global Brands LLC, Northfield, IL 60093 (US)
(72) Inventor: Brown, Peter Harris, Glenview, IL 60025 (US); Rubin, Martyn, Chicago, IL 60657 (US); Anderson, Gerald Harvery, Toronto Ontario M5R 1C6 (CA); Matusheski, Nathan V, Gurnee, IL 60031 (US); Laudano, Raymond J, Libertyville, IL 60048 (US); Kamp, Kyle Maxwell, Mundelein, IL 60060 (US); Desrochers, Julia Leigh, Arlington Heights, IL 60004 (US); McPherson, Andrew Edward, Mt. Prospect, IL 60056 (US)
(74) Representative: Murray, Adrian D'Coligny

(57) **Abstract**

A method is provided that is effective for reducing postprandial blood glucose levels. The method includes administering a blend of native whey protein and viscosifying fiber to a subject in an amount and at a time prior to a meal or concurrent with a meal that is effective for reducing postprandial blood glucose levels and/or reducing food intake. In a preferred embodiment, the viscosifying fibre is hydroxypropyl methylcellulose (MPMC) incorporated into a beverage formulation (Crytal Light™ drink mix) or bread.

## Description

This application claims the benefit of U.S. Provisional Application No. 61/093,037, filed August 29, 2008, which is incorporated in its entirety herein by reference.

The present invention relates to a method for reducing postprandial blood glucose levels. More particularly, the method includes administering a blend of native whey protein and viscosifying fiber to a subject in an amount and at a time prior to a meal or concurrently with a meal that is effective for reducing postprandial blood glucose levels.

### BACKGROUND

There is significant global growth of diabetes and pre-diabetes, the defining characteristic of which is chronic hyperglycemia. Following a meal, blood glucose levels in these individuals' and individuals at potential risk for diabetes increases beyond "normal" levels and remains high for a longer period of time. For diabetics, the previous solution has been the combination of drug therapy and a diet of low "glycemic" foods, that is, foods high in dietary fiber and low in fat and digestible carbohydrates (i.e., simple sugars and starch). For pre-diabetics and those who may be at risk for becoming diabetic by displaying postprandial hyperglycemia, the previous solution has been a diet of low glycemic foods.

### SUMMARY

A method is provided for controlling postprandial blood glucose levels by administration of specific amounts of a blend of whey protein and viscosifying fiber within a specific window of time prior to a meal or concurrently with a meal. Ingestion of a blend of whey protein and viscosifying fiber at certain times before a meal (i.e., a whey protein/ viscosifying fiber pre-load) or concurrently with a meal significantly reduces postprandial (after a meal) blood glucose levels and the kinetics of glucose appearance in the blood. The method is effective for maintaining a more normal blood glucose homeostasis.

More specifically, a method is provided for reducing postprandial blood glucose levels. The method includes administering a blend of native whey protein and viscosifying fiber to a subject. The blend includes from about 2 to about 50 grams, in another aspect about 2 to about 25 grams, in another aspect about 2 to about 10 grams, and in another aspect about 10 to about 25 grams of native whey protein. The blend further includes from about 0.5 to about 20 grams, in another aspect about 2 to about 20 grams, in another aspect about 0.5 to about 10 grams, and in another aspect about 2 to about 10 grams of viscosifying fiber. In this aspect, the blend includes a ratio of whey protein to viscosifying fiber of about 0.5 to 1 to about 12.5 to 1.

In an important aspect of the invention, native whey has a level of hydrolysis of about 2% or less. Native whey protein that may be utilized includes sweet whey, acid whey, individual whey proteins and mixture thereof.

Viscosifying fiber that may be utilized include hydroxypropyl methylcellulose, gum arabic, sodium carboxymethylcellulose, methylcellulose, guar gum, gellan gum, locust bean gum, konjac flour, tragacanth gum, karaya gum, gum acacia, chitosan, arabinoglactins, glucomannan, xanthan gum, alginate, pectin, low and high methoxy pectin, beta-glucans, carrageenan, psyllium and mixtures thereof. The blend of native whey protein and viscosifying fiber is administered to a subject such that the amount of viscosifying fiber administered with the native whey protein is 20 grams or less.

In one aspect, the blend of native whey protein and viscosifying fiber is administered to the subject at least about 5 to about 90 minutes, in another aspect about 20 to about 40 minutes, and preferably about 30 minutes prior to a meal. The method is effective for reducing postprandial blood glucose levels by at least about 5% as compared to the same subject not administered a blend of native whey protein and viscosifying fiber prior to a meal.

In another aspect, a method is provided for reducing postprandial blood glucose levels that includes administering an edible composition to a subject, where the edible composition includes a blend of whey protein and viscosifying fiber. The edible composition may include a beverage, a nutrition supplement, a food composition, a meal replacement product, and mixtures thereof. In this aspect, a food composition that includes 2 to 50 grams of native whey protein and 0.5 to 20 grams of viscosifying fiber may be administered at least about 5 to about 90 minutes prior to a meal or concurrently with a meal. The method is effective for reducing postprandial blood glucose levels by at least about 5% as compared to the same subject not administered a blend of native whey protein and viscosifying fiber prior to a meal or concurrently with a meal.

### BRIEF DESCRIPTION OF THE DRAWINGS

Figure 1 illustrates post-meal blood glucose iAUC (0-120 min) following consumption of a beverage containing protein and viscosifying fiber composition.

Figure 2 show post-meal blood insulin iAUC (0-120 min) following consumption of a beverage containing protein and viscosifying fiber composition.

Figure 3 illustrates post-meal glycemic index following consumption of a beverage containing protein and viscosifying fiber composition.

### DETAILED DESCRIPTION

A method is provided that is effective for reducing postprandial blood glucose levels. Accordingly, the method may also promote satiety, which may lead to weight loss and lowering of blood glucose levels. In accordance with the method, a blend of native whey protein and viscosifying fiber is administered to a subject directly or as part of an edible composition. Administration of the blend of native whey protein and viscosifying fiber is effective for reducing postprandial blood glucose levels by at least about 5%, preferably at least about 10% and most preferably at least about 20% as compared to the same subject not administered a blend of native whey protein and viscosifying fiber. In another aspect, the method is effective for reducing caloric intake by at least about 5%, preferably at least about 10%, and most preferably at least about 20% as compared to the same subjected not administered a blend of native whey protein and viscosifying fiber.

### Native Whey Protein

Native whey proteins that may be utilized include sweet whey, acid whey, individual whey proteins (i.e., α-lacalbumin, β-lactoglobulin, albumin, immunoglobulins, glycomaropeptide derived from casein, lactoferrin), and mixture thereof. Suitable commercial sources of native whey protein may be utilized. An example of a suitable commercial source of whey protein includes Alacen 392 whey protein concentrate (Fonterra) which is manufactured from fresh sweet cheese whey using an ultrafiltration process.

The native whey protein utilized has a very low level of hydrolysis. As used herein, "native whey protein" refers to whey protein that has a degree of hydrolysis of less than about 2%, in another aspect less than about 1%, and in another aspect, less than about 0.4% (as determined by OPA methodology, Lee et al. Int. J. Biochem. 1978; 9(7):457-467, which is incorporated herein by reference). Native whey protein may include whey protein concentrates, whey protein isolates, and hydrolysates of whey protein.

### Viscosifying Fiber

As used here herein, "viscosifying fiber" refers to dietary viscosifying fiber defined as "viscous fiber" and "viscous polysaccharide" in Dikeman CL, Fahey GC. Viscosity as related to dietary fiber: a review. Crit Rev Food Sci Nutr. 2006;46(8):649-63, which is incorporated herein by reference. Dietary viscosifying fiber is prepared in a form suitable for oral use according to any method known in the art for the manufacture of oral dietary viscosifying fiber compositions. Examples of dietary viscosifying fiber that may be used include hydroxypropyl methylcellulose, gum arabic, sodium carboxymethylcellulose, methylcellulose, guar gum, gellan gum, locust bean gum, konjac flour, tragacanth gum, karaya gum, gum acacia, chitosan, arabinogalactins, glucomannan, xanthan gum, alginate, pectin, low and high methoxy pectin, beta-glucans, carrageenan and psyllium. Numerous commercial sources of soluble dietary viscosifying fibers are readily available and known to one practicing the art. For example, gum arabic, hydrolyzed carboxymethylcellulose, guar gum, xanthan gum, alginates, pectin and the low and high methoxy pectins are available from TIC Gums, Inc. of Belcamp, Md. The oat and barley glucans are available from Mountain Lake Specialty Ingredients, Inc. of Omaha, Nebr. Psyllium is available from the Meer Corporation of North Bergen, N.J. while the carrageenan and konjac flour are available from FMC Corporation of Philadelphia, Pa.

### Edible Composition

The edible composition may be in the form of a nutritional supplement (such as a tablet, powder, capsule or liquid product), a food composition (product), a beverage, or a meal replacement product.

A nutritional supplement as used herein refers to a composition or supplement which provides at least one beneficial agent such as vitamins, minerals, trace elements, phytochemicals, which is intended to supplement the amount of such agents obtained through normal dietary intake. These compositions or supplements do not generally contain a significant amount of calories, protein, carbohydrate or fat. They are not intended to be taken as a food but rather as a supplement to the daily diet.

A food composition may be any food which can be formulated to include a blend of native whey protein and viscosifying fiber. Food compositions may include dairy based products (such as milk based products including hard and fresh cheese and yogurt), soy based products, breads and cereal based products (including pasta and cereal bars), cakes, cookies, biscuits, spreads, oil-in-water emulsions (such as dressings, ketchup and mayonnaise), ice creams, desserts, soups, powdered soup concentrates, sauces, powdered sauce concentrates, health bars, confectionery, snack foods, ready-to-eat meal products, pre-packed meal products, and dried meal products etc.

A beverage may be any beverage which can be formulated to include a blend of native whey protein and viscosifying fiber. Beverages may include water, fruit juice, a low calorie fruit flavored beverage (for example, Kool-Aid, Crystal Light, powdered and ready to drink soft drinks, sport drinks), coffee, tea, smoothie, a dairy beverage, a carbonated beverage, and mixtures thereof.

A meal replacement product as used herein refers to a product which is intended to replace one or more conventional meals a day; they are of a controlled calorie content and are generally eaten as a single product. However several such products may be eaten together. Examples of meal replacement products and products to be used as part of a meal replacement plan include; (ready-to-drink) liquid products such as milk or soy-based drinks, soluble powders used to prepare those drinks and drinks prepared therefrom, bars, soups, cereal or noodle or pasta-based products, desserts such as rice puddings, custards and the like and porridge and the like. Meal replacement products are generally used by consumers following a calorie controlled diet or wishing to control their body weight.

The edible composition may be for example; a solid product, a powdered product, a tablet, a capsule, a liquid, a flowable, spoonable, pourable or spreadable product or a bar etc. The edible composition may be a powder which is mixed with a liquid, such as water or milk, to produce a liquid or slurry product such as a meal replacement product, or a product to be used as part of a meal replacement plan.

In another aspect, the total amount of viscosifying fiber administered with the native whey protein is 20 grams or less. The total amount of viscosifying fiber administered in terms of a % of the edible composition will be less than about 50%, and in another aspect, less than about 20%, and in another aspect, less than about 2%.

### EXAMPLE 1: Beverage Formulation Containing Protein Combined with

### Viscosifying Fiber

Beverage ingredients selected based on taste and functionality (Table 1). An instantized whey protein concentrate 80% (WPC) with a clean flavor profile was combined with the viscosifying fiber hydroxypropyl methylcellulose (HPMC) at the indicated ratio. Crystal Light^{™} drink mix was used as a base to give the beverage flavor and color. Because of the functionality of ingredients antifoam was added to keep the functional ingredients evenly distributed in the liquid phase. The product was dry blended to achieve an even distribution of all ingredients. The product was analytically tested prior to administration in order to assure homogeneity.

**Table 1: Beverage formulation (expressed as amount given to subjects).**

| Ingredient | 5g WP¹ | 5g WP+2g HPMC² | Placebo |
|---|---|---|---|
| Whey protein concentrate | 6.3 | 6.2 | 0.0 |
| HPMC* | 0.0 | 2.0 | 0.0 |
| Crystal Light^{™} drink mix | 1.6 | 1.6 | 1.6 |
| Anti-foam | 0.0 | 0.0 | 0.0 |
| Lactose | 0.4 | 0.4 | 0.8 |
| Total | 8.3 | 10.1 | 2.4 |

| | | | |
|---|---|---|---|
| ¹WPC: Whey protein concentrate 80% ²HPMC: Hydroxypropyl methylcellulose | | | |

### EXAMPLE 2: Human Study Testing Effect of Beverage Formulated with Protein Combined with Viscosifying Fiber on Glycemic and Insulinemic Response

A randomized controlled crossover study was performed in human subjects to determine whether a glucose-lowering effect could be enhanced when whey protein is combined with hydroxypropyl methylcellulose (HPMC) viscosifying fiber. All treatments were served in liquid form (400 ml total) that included water, drink mix sweetened with nonnutritive sweetener for color and flavor, and the amounts of sweet whey protein indicated below (Example 1). Treatments were administered as preload (30 minutes prior to) or concurrent with the standard meal as described below.

**Treatment**

| No. | Treatment |
|---|---|
| 1 | Test beverage with 5 grams whey protein as preload |
| 2 | Test beverage with 5 grams whey protein + 2 g HPMC viscosifying fiber as preload |
| 3 | Placebo beverage (including drink mix) as preload (control) |
| 4 | Test beverage with 5 grams whey protein as concurrent |
| 5 | Test beverage with 5 grams whey protein + 2 g HPMC viscosifying fiber as concurrent |
| 6 | Placebo beverage (including drink mix) as concurrent (control) |

Treatment subjects included 21 males and females between 21 and 51 years old with a BMI between 25.0 and 30.0.

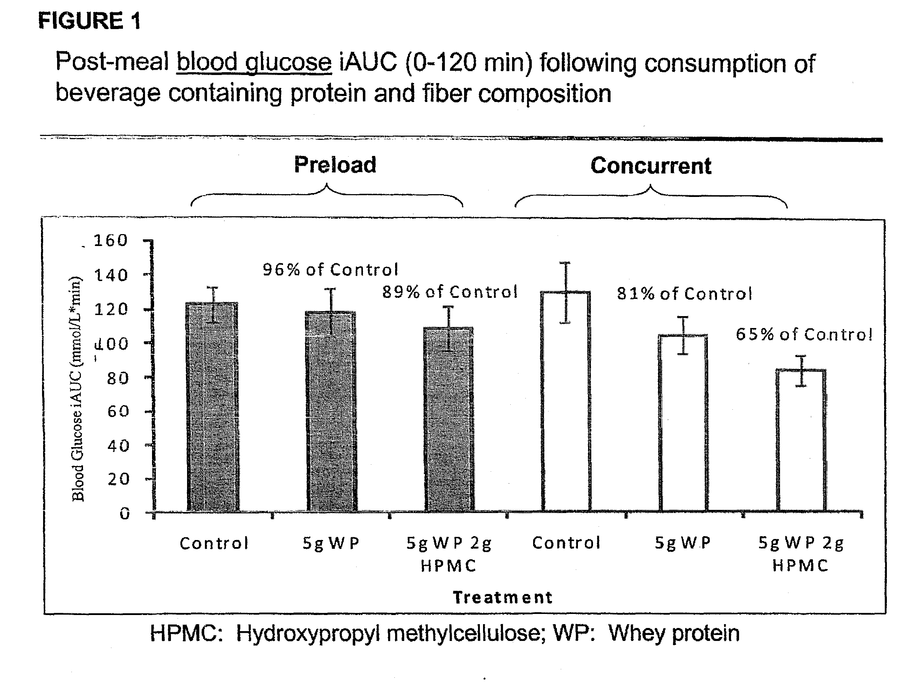

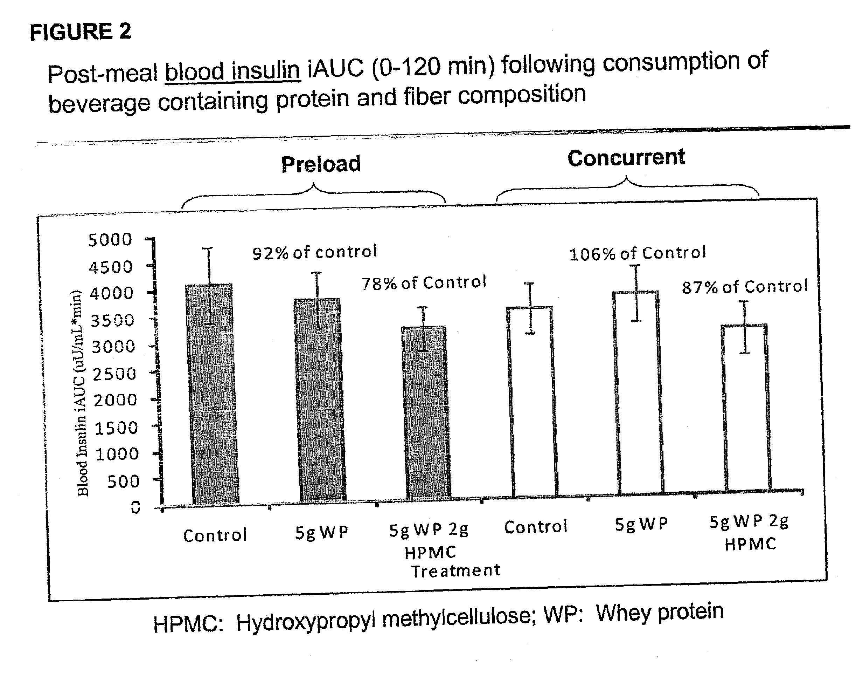

Treatment subjects randomly received the treatments after an overnight fast. Subjects were allowed 5 minutes to consume their assigned test beverage. Blood glucose and insulin by finger prick blood collection were measured immediately before and at 30 minutes after receiving the test beverage. Subjects then had 10 minutes to eat a standard meal providing 50 g of available carbohydrate. At the first bite of the standard meal a timer was started and additional finger-prick blood samples were taken at 15, 30, 45, 60, 90 and 120 min after the start of the meal. The effect of all treatments on food intake and blood glucose, and insulin are illustrated in Figures 1 and 2.

### EXAMPLE 3: Formulation of Bread Containing Protein Combined with Viscosifying Fiber

Bread ingredients were selected based on taste and functionality (Table 2). An instantized whey protein concentrate 80% (WPC) with a clean flavor profile was combined with the viscosity inducing viscosifying fiber hydroxypropyl methylcellulose (HPMC) at the indicated ratio. The functional ingredients were added to a modified bread dough formula to accommodate the additional functionality of the indicated ingredients. The formula changes allowed for normal processing and resulted in a baked bread product similar to control.

**Table 2: Bread Dough Formulation (expressed as 2 kg batch)**

| Ingredient | Control (g) | Test (g) |
|---|---|---|
| Flour | 1209 | 1046 |
| Water | 654 | 568 |
| WPC¹ | 0 | 200 |
| HPMC² | 0 | 64 |
| Sucrose | 36 | 31 |
| Soybean oil | 30 | 26 |
| Yeast | 24 | 21 |
| Salt | 15 | 13 |
| Mono and diglycerides | 12 | 10 |
| Emulsifier | 12 | 10 |
| Sodium Stearoyl Lactate | 4 | 4 |
| Bread Flavor | 3 | 3 |
| Calcium Propionate | 5 | 5 |
| Enzyme | 1 | 1 |
| Total | 2000.0 | 2000.0 |

| | | |
|---|---|---|
| ¹Whey protein concentrate 80% ²Hydroxypropyl methylcellulose | | |

### EXAMPLE 4: Glycemic Index Testing of Bread Formulated with Protein and Viscosifying Fiber

A glycemic index test was performed in human subjects to determine whether a glucose-lowering effect could be enhanced when whey protein is combined with hydroxypropyl methylcellulose (HPMC) viscosifying fiber in a formulated food product. All treatments were served in the form of baked bread, with the amounts of sweet whey protein and viscosifying fiber indicated below (Example 3).

**Treatment**

| No. | Treatment |
|---|---|
| 1 | Control bread |
| 2 | Test bread with 5 grams whey protein + 2 g HPMC viscosifying fiber |

Treatment subjects included 10 males and females between 46 and 59 years old with a BMI between 22 and 41.

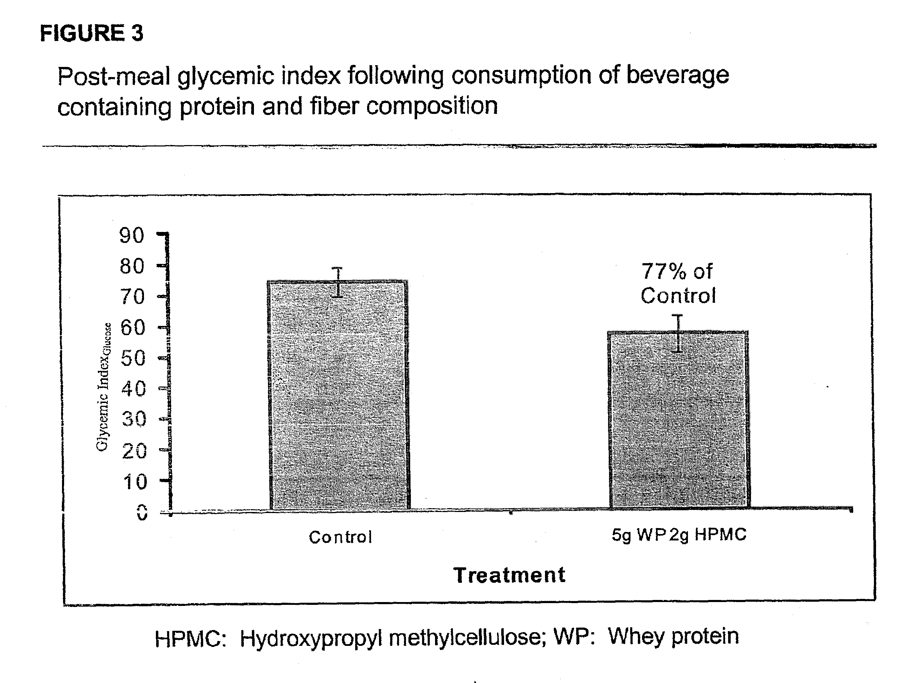

Glycemic index of the food products was determined following standard methods, and expressed relative to glucose. Subjects randomly received the treatments after an overnight fast. Subjects were allowed 10 minutes to consume their assigned test food, providing 25g of available carbohydrate (59.9g of control bread, 67.3g of test bread). At the first bite of the food a timer was started, and additional finger-prick blood samples were taken at 15, 30, 45, 60, 90 and 120 min after the start of the meal. A standardized white bread was used as a reference food (GI=71), and results of testing control and test products were expressed relative to glucose. The effect of treatments on glycemic index are illustrated in Figure 3.

## Claims

1. A method for reducing postprandial blood glucose levels comprising:
administering a blend of from 2 to 50 grams of native whey protein and 0.5 to 20 grams viscosifying fiber to a subject at least 5 to 90 minutes prior to a meal, the method effective for reducing postprandial blood glucose levels by at least about 5% as compared to the same subject not administered a blend of native whey protein and viscosifying fiber prior to a meal.

2. The method of claim 1 wherein the native whey protein has a level of hydrolysis of 2% or less.

3. The method of claim 1 or 2 wherein the viscosifying fiber is selected from the group consisting of hydroxypropyl methylcellulose, gum arabic, sodium carboxymethylcellulose methylcellulose, guar gum, gellan gum, locust bean gum, konjac flour, tragacanth gum, karaya gum, gum acacia, chitosan, arabinogalactins, glucomannan, xanthan gum, alginate, pectin, low and high methoxy pectin, beta glucans, carrageenan, psyllium and mixtures thereof.

4. The method of any one of Claims 1 to 3 wherein the blend of native whey protein and viscosifying fiber is administered about 20 to about 40 minutes prior to a meal.

5. A method for reducing postprandial blood glucose levels and/or food intake comprising:
administering an edible composition that includes a blend of from 2 to 50 grams of native whey protein and 0.5 to 20 grams of viscosifying fiber to a subject at least 5 to 90 minutes prior to a meal, the method effective for reducing postprandial blood glucose levels by at least about 5 % as compared to a subject not administered a blend of native whey protein and viscosifying fiber prior to a meal.

6. The method of Claim 5 wherein the viscosifying fiber is selected from the group consisting of hydroxypropyl methylcellulose, gum arabic, sodium carboxymethylcellulose, methylcellulose, guar gum, gellan gum, locust bean gum, konjac flour, tragacanth gum, karaya gum, gum acacia, chitosan, arabinoglactins, glucomannan, xanthan gum, alginate, pectin, low and high methoxy pectin, beta-glucans, carrageenan, psyllium and mixtures thereof.

7. The method of Claims 5 or 6 wherein the edible composition is administered about 20 to about 40 minutes prior to a meal.

8. A method for reducing postprandial blood glucose levels comprising:
administering an edible composition that includes from 2 to 50 grams of native whey protein and 0.5 to 20 grams of viscosifying fiber, the method effective for reducing postprandial blood glucose levels by at least about 5 % as compared to a subject not administered a food composition that includes native whey protein and viscosifying fiber.

9. The method of any one of Claims 5 to 8 wherein the edible composition that includes native whey protein is selected from the group consisting of a beverage, a nutritional supplement, a food composition, a meal replacement product, and mixtures thereof.

10. The method of Claim 9 wherein the beverage is selected from the group consisting of water, fruit juice, a low calorie fruit flavored beverage, coffee, tea, smoothie, a dairy beverage, a carbonated beverage, and mixtures thereof.

11. The method of Claim 9 wherein the nutritional supplement is selected from the group consisting of vitamins, minerals, trace elements, phytochemicals, and mixtures thereof.

12. The method of Claim 9 wherein the food composition is selected from the group consisting of dairy based products, soy based products, breads and cereal based products, cakes, cookies, biscuits, spreads, oil-in-water emulsions, ice creams, desserts, soups, powdered soup concentrates, sauces, powdered sauce concentrates, health bars, confectionery, snack foods, ready-to-eat meal products, pre-packed meal products, dried meal products, and mixtures thereof.

13. The method of any one of Claims 1 to 12 wherein the native whey protein is selected from the group consisting of sweet whey, acid whey, individual whey proteins, hydrolysates of native whey proteins, and mixture thereof.

14. The method of any one of Claims 1 to 13 wherein the native whey protein is administered in an amount of 2 to 25 grams per subject and the viscosifying fiber is administered in an amount of 0.5 to 20 grams per subject.
